# EUROPEAN PATENT SPECIFICATION

(11) **EP 1 618 894 B1**
(45) Date of publication and mention of the grant of the patent: **13.06.2018**
(21) Application number: 04729510.0
(22) Date of filing: 26.04.2004
(51) Int. Cl.: A61K 9/00, A61K 9/19, A61K 31/545, A61K 47/02, A61K 47/18

(54) **COMPOSITION FOR INJECTION**
ZUSAMMENSETZUNG ZUR INJEKTION
COMPOSITION POUR INJECTION

(30) Priority: 28.04.2003 JP 2003123329; 29.01.2004 JP 2004020742
(43) Date of publication of application: 25.01.2006
(73) Proprietor: Takeda Pharmaceutical Company Limited, Osaka-shi, Osaka 541-0045 (JP)
(72) Inventor: DOI, Yasuo, Yodogawa-ku, Osaka-shi, Osaka 532-0024 (JP); INOUE, Yuichiro, Hikari-shi, Yamaguchi 743-0011 (JP); TAKECHI, Nobuyuki, Hikari-shi, Yamaguchi 743-0011 (JP); SAKURAI, Yuusuke, Ibaraki-shi, Osaka 567-0895 (JP)
(74) Representative: dompatent von Kreisler Selting Werner - Partnerschaft von Patent- und Rechtsanwälten mbB
(86) International application number: PCT/JP2004/005984
(87) International publication number: WO 2004/096279

(56) References cited:
- EP-A- 0 319 856
- JP-A- 1 250 322
- JP-A- 3 090 019
- JP-A- 3 264 531
- JP-A- 4 338 332
- JP-A- 53 029 936
- JP-A- 2004 161 729
- US-A- 4 241 057
- US-A- 4 582 830

## Description

### Technical Field

The present invention relates to an injectable composition. More specifically, the present invention relates to an injectable composition comprising an acidic pharmaceutical compound and a basic substance.

### Background Art

Cephem antibiotics may be based on a salt or adduct with an acidic substance, which is advantageous in view of stability and manufacture in some instances. Examples of such cephem antibiotics include cefotiam hydrochloride (CTM), cefmenoxime hemihydrochloride (CMX) and cefozopran hydrochloride (CZOP), which are useful in clinical applications.

In addition, the compound represented by the following formula (I): 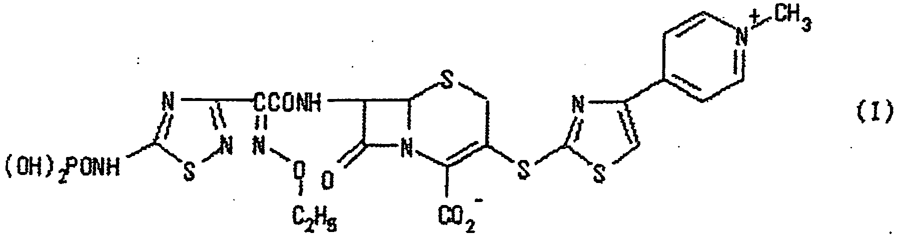 , which is effective against MRSA (methicillin resistant S. aureus) recently posing clinical problems, is also used as a salt or adduct thereof with an organic acid or inorganic acid.

An acidic pharmaceutical compound such as the acidic cephem antibiotics has undesirable properties in that it causes a pain felt by the patients at the time of injection. As an approach to resolve this problem, Japanese Unexamined Patent Application Publication No. 53-29936 discloses a composition containing cefotiam hydrochloride and sodium carbonate in an equivalent ratio of about 1:1 in Example 1. This reference also discloses a superior advantage of improved dissolution rate of CTM, which is afforded by the addition of sodium carbonate.

Generally, in dissolving an injection formulation, if the injection formulation is used under undesirable conditions generally not assumed (rapid increase in temperature), it may occur that the internal atmosphere of a vial increases, and a liquid leaks from a needle puncture site during suction of a dissolution liquid into a syringe. Even for the formulation combined with sodium carbonate, which is described in the above reference, if it is used in a vial formulation under undesirable conditions generally not assumed (rapid increase in temperature), the internal atmosphere of the vial excessively increases during dissolution, which may cause liquid leakage from the needle puncture site during suction of a dissolution liquid into a syringe, leaving room for improvement. There is thus a need to improve such problems in dissolving the products.

### Disclosure of Invention

In consideration of the above-mentioned problems, an object of the present invention is to provide an injectable composition free of liquid leakage from a needle puncture site during suction of a dissolution liquid into a syringe even if it is used under undesirable conditions generally not assumed (rapid increase in temperature).

The present inventors have investigated extensively to achieve the above-mentioned object, and have found that by combining an injectable composition comprising an acidic pharmaceutical compound and a basic compound other than carbonate, with the carbonate in a specific amount ratio, it is possible to obtain an injectable composition containing an acidic pharmaceutical compound free of excessive increase in the internal atmosphere of a vial even when it is used under undesirable conditions generally not assumed (rapid increase in temperature), and then have completed the present invention.

### Detailed Description of the Invention

The present invention will be described in detail below.

The acidic pharmaceutical compound used in the present invention is a pharmaceutical compound that is acidic when dissolved in purified water for injection. Such pharmaceutical compound is preferably acidic cephem antibiotic, which is acidic when dissolved in the purified water for injection, and the like. Examples of the acidic cephem antibiotics include a salt or adduct of cefotiam, cefmenoxime, cefozopran, cefpirome, cefepime, and a compound represented by the formula (I): 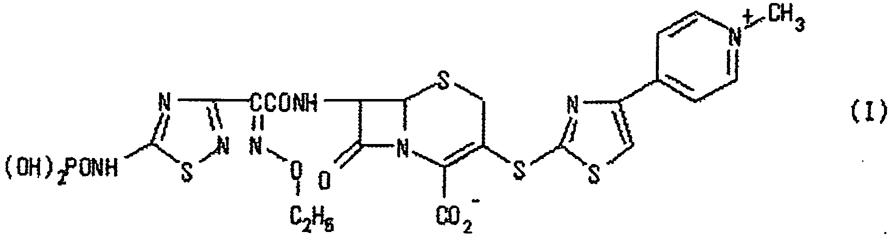 with an organic acid or an inorganic acid, or ceftazidime itself, which is an acidic substance.

The organic acid or inorganic acid is not particularly limited if it is pharmaceutically acceptable. Examples of the organic acid include formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, aspartic acid, glutamic acid, and the like. Examples of the inorganic acid include hydrochloric acid, hydrobromic acid, nitric acid, sulfuric acid, phosphoric acid, and the like. Hydrochloric acid, acetic acid, p-toluenesulfonic acid, and the like are preferred.

Preferred examples of the acidic cephem antibiotics include, specifically, cefotiam hydrochloride (CTM) represented by the formula (II): 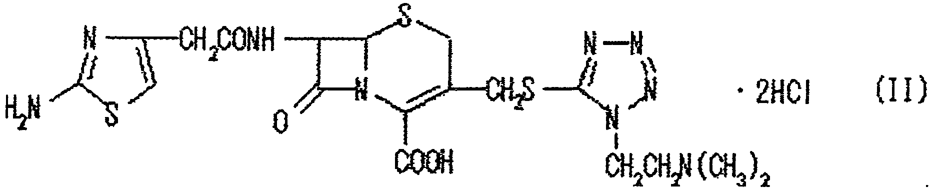 , cefmenoxime hemihydrochloride (CMX) represented by the formula (III): 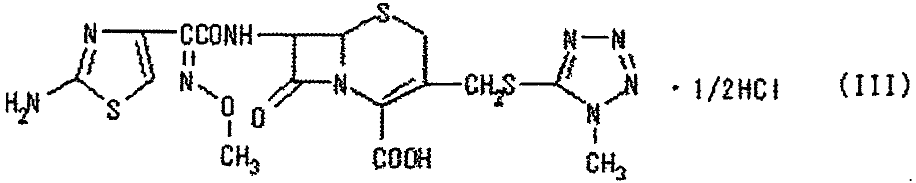 , cefozopran hydrochloride (CZOP) represented by the formula (IV) : 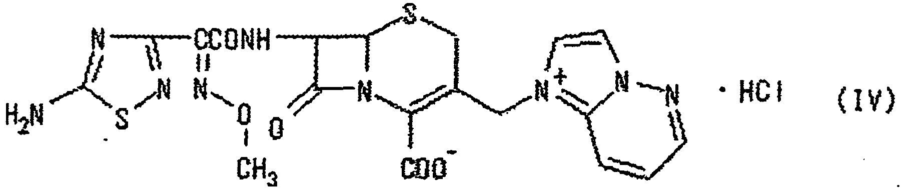 , cefpirome sulfate (CPR), ceftazidime (CAZ), cefepime dihydrochloride (CFPM), cefpirome sulfate, and acetate of a compound represented by the formula (I): 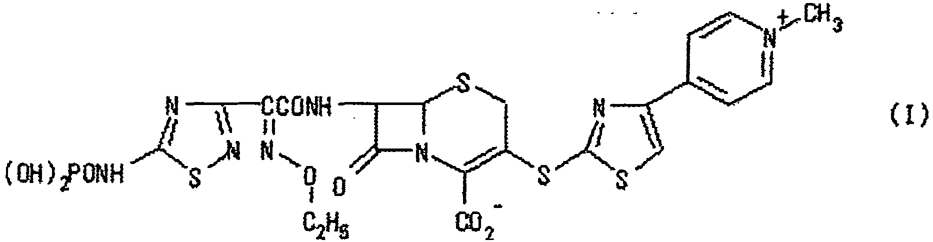 (hereinafter referred to simply as "acetate of Compound (I)").

These acidic cephem antibiotics may be used alone or in combination of 2 or more compounds.

Cefotiam, cefmenoxime, cefozopran, and the compound represented by the formula (I) can be prepared by a well-known method, or general organic synthesis. For example, they can be prepared by methods disclosed in publications below.

Cefotiam can be prepared, for example, using a method disclosed in Japanese Unexamined Patent Application Publication No. 53-29913 and a well-known general organic synthesis.

Cefmenoxime can be prepared, for example, using a method disclosed in Japanese Unexamined Patent Application Publication No. 55-79393 and a well-known general organic synthesis.

Cefozopran can be prepared, for example, using a method disclosed in Japanese Unexamined Patent Application Publication No. 1-250322 and a well-known general organic synthesis.

The compound represented by the formula (I) can be prepared, for example, using a method disclosed in Japanese Unexamined Patent Application Publication No. 11-255772 and any well-known general organic synthesis.

These compounds can be converted to salts or adducts with organic acids or inorganic acids by any well-known method or the methods disclosed in the above-mentioned publications.

These antibiotics or salts or adducts thereof with an organic acid or inorganic acid may be also solvent adducts (solvate) or non-solvent adducts (non-solvates), particularly hydrates or non-hydrates. In addition, when these compounds exist as configurational isomers, diastereomers, conformers, and the like, it is possible to isolate individual isomers, if desired, using any well-known separation and purification method. When these compounds are racemates, they can be separated into S- and R-isomers by means of a conventional optical resolution method. When stereoisomers of these compounds exist, individual stereoisomers and a mixture thereof are also disclosed. Further, these compounds may be labeled with an isotope (for example, ³H, ¹⁴C and ³⁵S), and the like.

The carbonate used in the present invention is sodium carbonate. This carbonate may be used alone or in combination of 2 or more compounds.

The basic compounds other than the carbonate include (1) organic bases such as basic amino acids, and (2) inorganic bases other than carbonates. Examples of the basic amino acids include arginine, lysine, histidine, ornithine and the like. Among these, arginine is preferred. Either the D-isomer or L-isomer may be used, but the L-isomer is preferred. Examples of the inorganic bases other than the carbonates include trisodium phosphate, disodium phosphate, sodium hydroxide, meglumine and the like. Among these, trisodium phosphate is preferred. These basic compounds may be used alone or in combination of 2 or more compounds.

In addition, the injectable composition may contain a buffer (for example, sodium dihydrogen phosphate, disodium hydrogen phosphate and the like), an isotonizing agent (for example, glucose, sodium chloride and the like), a stabilizer (for example, sodium hydrogen sulfite and the like), a soothing agent (for example, glucose, benzyl alcohol, mepivacaine hydrochloride, xylocaine hydrochloride, procaine hydrochloride, carbocaine hydrochloride and the like), a preservative (for example, p-oxybenzoic acid ester such as methyl p-oxybenzoate and the like, thimerosal, chlorobutanol, benzyl alcohol and the like) and the like, if necessary.

In addition, the injectable composition may contain vitamins and the like.

Further, the injectable composition of the present invention may contain an aqueous solvent, if necessary. Examples of the aqueous solvent include purified water for injection, physiological saline solution, and glucose solution.

In the injectable composition of the present invention, at least the acidic pharmaceutical compound is solid. The carbonate and the basic compound other than the carbonate may be solid, respectively, or may be a liquid such as a solution dissolved in the above-mentioned aqueous solvent. Preferably, all of these 3 components are solid. As used herein, the "solid" comprises crystals and amorphous substances which have conventional meanings. The form of the solid component is not particularly limited, but powder is preferred in view of dissolution rate.

The injectable composition of the present invention is characterized in that a small amount of carbon dioxide is generated when the carbonate and the acidic pharmaceutical compound are mixed in an aqueous solvent, but bubbles disappear rapidly. In this regard, the solid component is surprisingly rapidly dissolved although the bubbles generate in a small amount. In addition, the generated bubbles rapidly disappear, and thereby enabling rapid visual confirmation of dissolution of the solid component.

As used herein, "mixing the carbonate and the acidic pharmaceutical compound in an aqueous solvent" refers to, for example, (1) mixing a solid comprising the carbonate and the acidic pharmaceutical compound, with an aqueous solvent, and (2) mixing a solid of the acidic pharmaceutical compound with an aqueous solution containing the carbonate, and the like. In other words, the aqueous solvent may be prepared separately, or incorporated into the composition of the present invention.

In this regard, the basic compound other than the carbonate may or may not be mixed simultaneously, but is preferably mixed simultaneously.

As used herein, "a small amount (of generated carbon dioxide)" means that, for example, (1) when the composition of the present invention is a vial formulation, the amount of generated carbon dioxide is small to the extent that no liquid leakage occurs in sucking an injectable solution into a syringe, though the internal atmosphere of the vial is not reduced previously to a near vacuum level (specifically, the internal atmosphere of 13.3 kPa or less), or (2) when the injectable composition of the present invention is connected to a general infusion set (for example, Terufusion infusion set (trademark), Terumo Corporation) as a vial formulation or a kit formulation comprising a bag, and continuous infusion operation is carried out, the amount of the carbon dioxide generated is small to the extent that decrease in the liquid level of the infusion tube is 10 mm or less (more preferably 7 mm or less, particularly preferably 5 mm or less, and most preferably 3 mm or less) using a glucose solution as a comparative control.

More specifically, "an injectable composition, wherein a small amount of the carbon dioxide is generated" in the present specification is, for example, an injectable composition wherein the internal atmosphere of a vial after dissolution is about 90 to about 140 kPa when 1 g (potency) of a solid component of the injectable composition of the present invention is dissolved in a 35 mL vial of about 77 kPa of internal atmosphere at room temperature to prepare 5 mL of an injectable solution.

The amount of the carbon dioxide generated can be controlled by adjusting the combination equivalent (or the combination amount) of the carbonate.

The combination equivalent (or combination amount) of the carbonate varies with the type of acidic pharmaceutical compounds and the like. The combination equivalent of the carbonate is usually about 0.1 to about 3.4 equivalents, preferably about 0.2 to about 3.0 equivalents, more preferably about 0.3 to about 2.0 equivalents, and further preferably about 0.4 to about 1.3 equivalents, per 1 equivalent of the acidic pharmaceutical compound. More specifically, for example, when the acidic pharmaceutical compound is cefotiam hydrochloride, the combination equivalent of the carbonate is usually about 0.1 to about 1.0 equivalent, preferably about 0.2 to about 0.7 equivalents, and more preferably about 0.3 to about 0.6 equivalents, per 1 equivalent of cefotiam hydrochloride, and when the acidic pharmaceutical compound is acetate of Compound (I), the combination equivalent of the carbonate is usually about 0.3 to about 3.4 equivalents, preferably about 0.6 to about 2.3 equivalents, and more preferably about 1.0 to about 2.0 equivalents, per 1 equivalent of the acetate of Compound (I). On the other hand, the combination amount of the carbonate is usually about 0.01 to about 0.22 parts by weight, preferably about 0.03 to about 0.20 parts by weight, more preferably about 0.05 to about 0.13 parts by weight, and further preferably about 0.06 to 0.09 parts by weight, per 1 part by weight of the acidic pharmaceutical compound. More specifically, for example, when the acidic pharmaceutical compound is cefotiam hydrochloride, the combination amount of the carbonate is usually about 0.01 to about 0.17 parts by weight, preferably about 0.03 to about 0.12 parts by weight, and more preferably about 0.05 to about 0.10 parts by weight, per 1 part by weight of cefotiam hydrochloride, and when the acidic pharmaceutical compound is acetate of Compound (I), the combination amount of the carbonate is usually about 0.02 to about 0.22 parts by weight, preferably about 0.03 to about 0.15 parts by weight, and more preferably about 0.06 to about 0.13 parts by weight, per 1 part by weight of the acetate of Compound (I).

The pH of an injection formulation containing the dissolved composition of the present invention is preferably about 3.5 to about 9.5, more preferably about 4.5 to about 9, particularly preferably about 5.5 to about 8.5, and most preferably about 6 to about 8 in view of inhibition of the pain associated with the injection.

The pH can be easily controlled by adjusting the combination equivalents (or combination amounts) of the carbonate and the basic compound other than the carbonate.

In other words, the sum of combination equivalents (or combination amounts) of the carbonate and the basic compound other than the carbonate (hereinafter referred to simply as "total amount of basic compound") in the injectable composition of the present invention varies with the type of the acidic pharmaceutical compound and the like, in view of pH adjustment. However, the total amount (combination equivalent) of basic compound is usually about 0.3 to about 6.2 equivalents, preferably about 0.6 to about 5.7 equivalents, more preferably about 0.9 to about 4.6 equivalents, and further preferably about 1.5 to about 3.8 equivalents, per 1 equivalent of the acidic pharmaceutical compound. More specifically, for example, when the acidic pharmaceutical compound is cefotiam hydrochloride, the total amount (combination equivalent) of basic compound is usually about 0.3 to about 3.0 equivalents, preferably about 0.6 to about 2.6 equivalents, and more preferably about 1.4 to about 2.4 equivalents, per 1 equivalent of cefotiam hydrochloride, and when the acidic pharmaceutical compound is acetate of Compound (I), the total amount (combination equivalent) of basic compound is usually about 0.6 to about 6.2 equivalents, preferably about 1.1 to about 5.0 equivalents, and more preferably about 2.6 to about 4.6 equivalents, per 1 equivalent of the acetate of Compound (I). On the other hand, the total amount (combination amount) of basic compound is usually about 0.07 to about 0.82 parts by weight, preferably about 0.14 to about 0.77 parts by weight, more preferably about 0.25 to about 0.69 parts by weight, and further preferably about 0.36 to about 0.64 parts by weight, per 1 part by weight of the acidic pharmaceutical compound. More specifically, for example, when the acidic pharmaceutical compound is cefotiam hydrochloride, the total amount (combination amount) of basic compound is usually about 0.07 to about 0.72 parts by weight, preferably about 0.14 to about 0.64 parts by weight, and more preferably about 0.35 to about 0.56 parts by weight, per 1 part by weight of cefotiam hydrochloride, and when the acidic pharmaceutical compound is acetate of Compound (I), the total amount (combination amount) of basic compound is usually about 0.08 to about 0.82 parts by weight, preferably about 0.14 to about 0.71 parts by weight, and more preferably about 0.39 to about 0.68 parts by weight, per 1 part by weight of the acetate of Compound (I).

The combination equivalent (or combination amount) of the basic compound other than the carbonate in the injectable composition of the present invention is determined based on the carbonate combined in the amount as described, the desired pH and the total amount of basic compound. Specifically, the combination equivalent (or combination amount) of the basic compound other than the carbonate varies with the type of the acidic pharmaceutical compound and the like. The combination equivalent of the basic compound other than the carbonate may be usually about 0.2 to about 2.8 equivalents, preferably about 0.4 to about 2.7 equivalents, more preferably about 0.6 to about 2.6 equivalents, and further preferably about 1.1 to about 2.5 equivalents, per 1 equivalent of the acidic pharmaceutical compound. More specifically, for example, when the acidic pharmaceutical compound is cefotiam hydrochloride, the combination equivalent of the basic compound other than the carbonate is usually about 0.2 to about 2.0 equivalents, preferably about 0.4 to about 1.9 equivalents, and more preferably about 1.1 to about 1.8 equivalents, per 1 equivalent of cefotiam hydrochloride, and when the acidic pharmaceutical compound is the acetate of Compound (I), the combination equivalent of the basic compound other than the carbonate is usually about 0.3 to about 2.8 equivalents, preferably about 0.5 to about 2.7 equivalents, more preferably about 1.6 to about 2.6 equivalents, per 1 equivalent of the acetate of Compound (I). On the other hand, the combination amount of the basic compound other than the carbonate is usually about 0.06 to about 0.60 parts by weight, preferably about 0.11 to about 0.57 parts by weight, more preferably about 0.20 to about 0.56 parts by weight, and further preferably about 0.30 to about 0.55 parts by weight, per 1 part by weight of the acidic pharmaceutical compound. More specifically, for example, when the acidic pharmaceutical compound is cefotiam hydrochloride, the combination amount of the basic compound other than the carbonate is usually about 0.06 to about 0.55 parts by weight, preferably about 0.11 to about 0.52 parts by weight, and more preferably about 0.30 to about 0.46 parts by weight, per 1 part by weight of cefotiam hydrochloride, and when the acidic pharmaceutical compound is acetate of Compound (I), the combination amount of the basic compound other than the carbonate is usually about 0.06 to about 0.60 parts by weight, preferably about 0.11 to about 0.56 parts by weight, and more preferably about 0.33 to about 0.55 parts by weight, per 1 part by weight of the acetate of Compound (I).

The composition of the present invention may be in various forms such as a vial formulation and a kit formulation.

Examples of the forms include:
Form 1: a solid containing the acidic pharmaceutical compound, the carbonate and a basic compound other than the carbonate (for example, powder, granule and the like);
Form 2: a vial formulation in which the solid of Form 1 is encapsulated;
Form 3: a kit formulation comprising the solid of Form 1 and an aqueous solvent; and
Form 4: a kit formulation comprising the solid, which contains the acidic pharmaceutical compound and the basic compound other than the carbonate, and a solution of the carbonate; and the like.

When the composition of the present invention is a vial formulation (preferably, a vial formulation of Form 2), the vial size is suitably selected according to the use of the vial. For example, when the aqueous solvent is poured into a vial and the solid component of the injectable composition is dissolved in the solvent to prepare an injectable solution, and then the injectable solution is used by sucking into a syringe, the vial size is about 3 to about 50 mL, preferably about 5 to about 45 mL, and more preferably about 5 to about 40 mL. The internal atmosphere of the vial is preferably about 90 to about 140 kPa when the aqueous solvent is poured into a vial and the solid component of the injectable composition is dissolved in the solvent to prepare an injectable solution, in view of prevention of its leakage during sucking into the syringe and ease of sucking into the syringe.

On this account, the internal atmosphere of the vial before use at room temperature (25°C) is preferably about 40 to about 100 kPa, more preferably about 50 to about 90 kPa, most preferably about 75 to about 85 kPa. In addition, the amount of the acidic pharmaceutical compound in this case is, for example, preferably 0.2 to 5 g, more preferably 0.5 to 2 g for a vial of 35 mL, preferably 0.2 to 4 g, more preferably 0.25 to 1 g, for a 17 mL vial, and preferably 0.2 to 4 g, more preferably 0.1 to 0.5 g, for a 9 mL vial.

When a vial is used for infusion, a large amount of injectable solution needs to be prepared at a time, and the vial size is, for example, about 51 to about 300 mL, preferably about 70 to about 200 mL, more preferably about 90 to about 150 mL. When a solution for infusion is prepared by pouring an aqueous solvent into the vial and dissolving the solid component of the injectable composition in the solvent, the internal atmosphere of the vial is preferably about 90 to about 110 kPa, in view of prevention of its leakage during sucking into the syringe and ease of sucking into the infusion vessel (for example, 130 mL vial and the like).

On this account, the internal atmosphere of the vial before use at room temperature (25°C) is preferably about 0.5 to about 39 kPa, more preferably 0.7 to about 20 kPa, and particularly preferably about 1 to about 10 kPa. In addition, the amount of the acidic pharmaceutical compound in this case is, for example, preferably 2 to 8 g, more preferably 3 to 6 g for a 300 mL vial, preferably 1 to 6 g, more preferably 2 to 4 g for a 200 mL vial, and preferably 0.2 to 5 g, more preferably 1 to 3 g for a 100 mL vial.

Suitable examples of a kit formulation of Form 3 include a kit formulation which comprises a bag consisting of two integrally-formed compartments divided by a partition wall, in which a solid containing the acidic pharmaceutical compound, a carbonate and a basic compound other than the carbonate are encapsulated in a compartment, a physiological saline solution or glucose solution as a dissolution liquid is encapsulated in the other compartment, and the partition wall is constructed in such a manner that the two compartments communicate with each other easily at use in order to mix and dissolve the solid and the dissolution liquid easily at use; a kit formulation which is a combination of the vial formulation of Form 2 and a half kit of an aqueous solvent; and the like.

The amount of the aqueous solvent in Form 3 and the solution in Form 4 is not particularly limited, is usually about 50 mL to about 200 mL, and is preferably about 80 to about 120 mL per 1 g of the acidic pharmaceutical compound.

The composition of the present invention can be prepared by employing a conventional method corresponding to the form.

For example, the solid of Form 1 can be prepared by pulverizing each compound using any well-known conventional mill (for example, power mill, jet mill and the like) to obtain a powder, and blending the powder with the acidic pharmaceutical compound using any well-known conventional mixer (for example, cross mixer, rocking mixer and the like). In particular, the basic compound other than the carbonate has a high hygroscopicity, and thus the stability of products may be degraded when it absorbs moisture and has a high water content. Therefore, the powder is preferably obtained by milling under a low-humidity environment which is not more than the equilibrium relative humidity of the basic compound other than the carbonate.

Regarding the particle size of the acidic pharmaceutical compound in the injectable composition of the present invention, for example, the particle size (median size, particle size distribution based on volume) of acidic cephem antibiotics is preferably about 200 µm or less and more preferably about 150 µm or less, in view of dissolution rate and easy handling. In addition, the particle size (median size, particle size distribution based on volume) of the basic compound other than the carbonate is preferably about 250 µm or less, and more preferably about 200 µm or less. Further, the particle size (median size, particle size distribution based on volume) of the carbonate is preferably about 250 µm or less, and more preferably about 200 µm or less.

A solution of the injectable composition of the present invention as described above, an injectable composition obtained by lyophilizing the solution, and an injectable composition obtained by freezing the solution are among the forms of the injectable composition of the present invention, and they are within the scope of the present invention.

The injectable composition of the present invention can be formulated into an injectable solution by any conventional method corresponding to the form of the composition, and can be administered to patients.

In the present invention, the dose of the salt or adduct of 1 or more compounds selected from the group consisting of cefotiam, cefmenoxime, cefozopran and a compound represented by the formula (I) with an organic acid or inorganic acid varies with the type of the compounds, symptoms and the like, but, for example, it is possible to administer 0.1 to 4 g (potency) once or 2 to 4 times daily.

According to the present invention, it is possible to provide an injectable composition containing acidic cephem antibiotics, which is free of an excessive increase in the internal atmosphere of a vial and a decrease in the liquid level in an infusion tube even when it is used under undesirable conditions generally not assumed (rapid increase in temperature), wherein each component dissolves rapidly, and further, dissolution of solid components can be quickly confirmed visually.

### EXAMPLES

The present invention will be described in more detail below with reference to the following Examples and Experimental Example, which are not intended to limit the present invention.

Milling of L-arginine used in Examples 1, 2, 3, and 4 was carried out under an environment with humidity no more than the ERH (Equilibrium Relative Humidity) of L-arginine (30% RH or less). A power mill (manufactured by Showa Chemical Industry Co., Ltd.) was used for coarse-milling, and a jet mill (manufactured by Nippon Pneumatic Mfg Co., Ltd.) was used for fine-milling.

The particle size (median size) of the powder of milled L-arginine and the like was measured using a laser diffraction type particle-size distribution measuring instrument (manufactured by HEROS & RODOS SYMPATEC Co.) according to the method below. In the present specification, the particle size (median size) refers to the values obtained using this method.

A method of measuring particle size (median size)

Particle size distribution was measured using a wet-type measuring method (CUVETTE dispersion) in which milled L-arginine and the like were dispersed in a suitable dispersion medium (for example, in case of L-arginine, saturated ethanol solution of L-arginine), respectively. From the obtained particle size distribution based on volume, the particle size (median size) was read.

### Example 1

1 g (potency) of cefotiam hydrochloride of a median size of about 120 µm, 81 mg of sodium carbonate of a median size of about 100 µm and 508 mg of L-arginine of a median size of about 170 µm were filled into a glass vial of an inner capacity of 35 mL, and a rubber stopper was inserted under reduced pressure of about 77 kPa to prepare a vial product. To this vial was added about 5 mL of purified water for injection, and the vial was shaken lightly to rapidly dissolve the contents.

### Example 2

To 100 g (potency) of cefotiam hydrochloride of a median size of about 120 µm was added 8.1 g of sodium carbonate of a median size of about 20 µm, and this mixture was mixed thoroughly using a mixer. Into a glass vial of an inner capacity of 35 mL were filled an amount corresponding to 1 g (potency) of cefotiam hydrochloride of the mixed powder, and 508 mg of L-arginine of a median size of about 170 µm, and a rubber stopper was inserted under reduced pressure of about 77 kPa to prepare a vial product. To this vial was added about 5 mL of purified water for injection, and the vial was shaken lightly to rapidly dissolve the contents.

### Example 3

To 1 kg (potency) of cefotiam hydrochloride of a median size of about 120 µm was added 81 g of sodium carbonate of a median size of about 20 µm, and this mixture was mixed thoroughly using a mixer. Into a glass vial of an inner capacity of 35 mL were filled an amount corresponding to 1 g (potency) of cefotiam hydrochloride of the mixed powder, and 508 mg of L-arginine of a median size of about 170 µm, and a rubber stopper was inserted under reduced pressure of about 77 kPa to prepare a vial product. This vial was connected with a half kit of a physiological saline solution (100 mL), about 10 mL of the physiological saline solution was transferred into the vial, and the vial was shaken lightly to rapidly dissolve the contents. The whole quantity of the contents was transferred into a plastic bottle of the half kit, and the vial and a double-ended needle were pulled out. An infusion set was set to the plastic bottle, and infusion operation was carried out under the conditions of infusion height of about 1 m and infusion time of 2 hours. As a result, a decrease in the liquid level of the infusion tube scarcely occurred.

### Example 4

To 1 kg (potency) of cefotiam hydrochloride of a median size of about 120 µm was added 81 g of sodium carbonate of a median size of about 20 µm, and this mixture was mixed thoroughly using a mixer. To this mixed powder was added 508 g of L-arginine of a median size of about 170 µm, and this mixture was mixed thoroughly using a mixer. Into a glass vial of an inner capacity of 35 mL was filled an amount corresponding to 1 g (potency) of cefotiam hydrochloride of the mixed powder and a rubber stopper was inserted under reduced pressure of about 77 kPa to prepare a vial product. This vial was connected with a half kit of a physiological saline solution (100 mL) which had been stored at 5°C, about 10 mL of the physiological saline solution was transferred into the vial, and the vial was shaken lightly to rapidly dissolve the contents. The whole quantity of the contents was transferred into a plastic bottle of the half kit, and the vial and the double-ended needle were pulled out. An infusion set was set to the plastic bottle, and infusion operation was carried out under the conditions of infusion height of about 1 m and infusion time of 2 hours under environment of 40°C. As a result, the liquid level of the infusion tube was 3 mm, which had little difference with that obtained when infusion operation was carried out with a physiological saline solution alone.

### Example 5

To 500 g (potency) of cefotiam hydrochloride of a median size of about 120 µm was added 300 g of sodium carbonate of a median size of about 20 µm, and this mixture was mixed thoroughly using a mixer. Into a glass vial of an inner capacity of 35 mL were filled an amount corresponding to 1 g (potency) of cefotiam hydrochloride of the mixed powder and 580 mg of L-arginine of a median size of about 170 µm, and a rubber stopper was inserted under reduced pressure of about 77 kPa to prepare a vial product. To this vial was added about 5 mL of purified water for injection, and the vial was shaken lightly to rapidly dissolve the contents.

### Example 6

To 1 kg (potency) of cefotiam hydrochloride of a median size of about 120 µm was added 113.9 g of sodium carbonate of a median size of about 20 µm, and this mixture was mixed thoroughly using a mixer. To this mixed powder was added 457.5 g of L-arginine of a median size of about 35 µm, and this mixture was mixed thoroughly using a mixer. An amount corresponding to 1 g (potency) of cefotiam hydrochloride of the mixed powder was filled into one of two compartments in a soft bag wherein the two compartments are separated by a partition wall and can communicate with each other by easy manipulation, and the other compartment was filled previously with a physiological saline solution, to prepare a bag product. By pushing the dissolution liquid side of this bag to open the partition wall, and conducting a pumping operation 2 or 3 times, the contents were rapidly dissolved. An infusion set was set to the bag, and infusion operation was carried out under the condition of infusion height of about 1 m and infusion time of 1 hour. As a result, a decrease in the liquid level of the infusion tube scarcely occurred.

### Example 7

To 1 kg (potency) of cefotiam hydrochloride of a median size of about 120 µm was added 113.9 g of sodium carbonate of a median size of about 20 µm, and this mixture was mixed thoroughly using a mixer. To this mixed powder was added 457.5 g of L-arginine of a median size of about 35 µm, and this mixture was mixed thoroughly using a mixer. Into a glass vial of an inner capacity of 35 mL was filled an amount corresponding to 1 g (potency) of cefotiam hydrochloride of the mixed powder, and a rubber stopper was inserted under reduced pressure of about 85 kPa to prepare a vial product. To this vial was added about 20 mL of purified water for injection, and the vial was shaken lightly to rapidly dissolve the contents.

### Example 8

1 g (potency) of acetate of Compound (I), 123.8 mg of sodium carbonate, 458 mg of L-arginine and 18.4 mg of sodium sulfite were filled into a glass vial of an inner capacity of 35 mL, and a rubber stopper was inserted under reduced pressure of about 77 kPa to prepare a vial product. To this vial was added about 10 mL of purified water for injection, and the vial was shaken lightly to rapidly dissolve the contents.

### Example 9

To 100 g (potency) of acetate of Compound (I) was added 12.38 g of sodium carbonate, and this mixture was mixed thoroughly using a mixer. Into a glass vial of an inner capacity of 35 mL were filled an amount corresponding to 1 g (potency) of acetate of Compound (I) of the mixed powder, 458 mg of L-arginine and 18.4 mg of sodium sulfite, and a rubber stopper was inserted under reduced pressure of about 77 kPa to prepare a vial product. To this vial was added about 10 mL of purified water for injection, and the vial was shaken lightly to rapidly dissolve the contents.

### Example 10

Into a glass vial of an inner capacity of 35 mL was filled 1 g (potency) of acetate of Compound (I), and a rubber stopper was inserted under reduced pressure of about 77 kPa to prepare a vial product.

123.8 mg of sodium carbonate, 458 mg of L-arginine and 18.4 mg of sodium sulfite were dissolved in 10 mL of distilled water and this mixture was filled into an ampoule. The space portion was substituted with nitrogen gas and the ampoule was melt-sealed to prepare a dedicated dissolution liquid.

To the vial product was added the dedicated dissolution liquid, and the vial was shaken lightly to rapidly dissolve the contents.

### Example 11

To 100 g (potency) of acetate of Compound (I) was added 12.38 g of sodium carbonate, and this mixture was mixed thoroughly using a mixer. Into a glass vial of an inner capacity of 35 mL was filled an amount corresponding to 1 g (potency) of acetate of Compound (I) of the mixed powder, and a rubber stopper was inserted under reduced pressure of about 77 kPa to prepare a vial product.

458 mg of L-arginine and 18.4 mg of sodium sulfite were dissolved in 10 mL of distilled water and the mixture was filled into an ampoule. The space portion was substituted with nitrogen gas and the ampoule was melt-sealed to prepare a dedicated dissolution liquid.

To the vial product was added the dedicated dissolution liquid, and the vial was shaken lightly to rapidly dissolve the contents.

### Experimental Example 1

A vial product was prepared in the same manner as in Example 1 except that the internal atmosphere was adjusted to 77 kPa. The temperature of this product was adjusted to 25°C. About 5 mL of purified water for injection of the same temperature was added by a syringe. The vial was shaken lightly to completely dissolve the contents. The internal atmosphere was measured, and as a result, found to be 109 kPa. The solution was sucked out with a syringe, and as a result, no leakage of the solution occurred, permitting easy sucking out of the solution.

### Industrial Applicability

According to the present invention, it is possible to provide an injectable composition comprising an acidic pharmaceutical compound, which is free of an excessive increase in the internal atmosphere of a vial and a decrease in the liquid level in an infusion tube even when it is used under undesirable conditions generally not assumed (rapid increase in temperature), wherein each component dissolves rapidly, and further, dissolution of solid components can be quickly confirmed visually.

## Claims

1. An injectable composition comprising an acidic pharmaceutical compound, sodium carbonate and arginine, wherein at least the acidic pharmaceutical compound is solid, wherein the acidic pharmaceutical compound is an organic acid salt, an inorganic acid salt, an organic acid adduct or an inorganic acid adduct of cefotiam, or a compound represented by the formula (I): 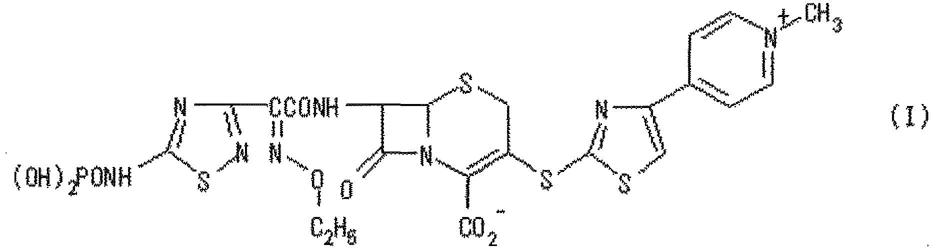 wherein the total amount of sodium carbonate and arginine is 0.3 to 6.2 equivalents per 1 equivalent of the acidic pharmaceutical compound,
wherein the amount of sodium carbonate is 0.1 to 3.4 equivalents per 1 equivalent of the acidic pharmaceutical compound.

2. The injectable composition according to Claim 1, wherein the acidic pharmaceutical compound is cefotiam hydrochloride.

3. The injectable composition according to Claim 1, wherein pH after dissolution is 3.5 to 9.5.

4. The injectable composition according to Claim 1, wherein the total amount of sodium carbonate and arginine is 0.07 to 0.82 parts by weight per 1 part by weight of the acidic pharmaceutical compound.

5. The injectable composition according to Claim 1, wherein the amount of sodium carbonate is 0.01 to 0.22 parts by weight per 1 part by weight of the acidic pharmaceutical compound.

6. The injectable composition according to Claim 1, wherein the amount of arginine is 0.2 to 2.8 equivalents per 1 equivalent of the acidic pharmaceutical compound.

7. The injectable composition according to Claim 1, wherein the amount of arginine is 0.06 to 0.60 parts by weight per 1 part by weight of the acidic pharmaceutical compound.

8. A vial formulation containing the injectable composition according to Claim 1.

9. The vial formulation according to Claim 8, wherein the internal atmosphere at 25°C is 40 to 100 kPa.

10. A kit formulation containing the injectable composition according to Claim 1.

11. The kit formulation according to Claim 10, comprising a 3 to 50 mL vial containing the injectable composition according to Claim 1, wherein the internal atmosphere of the vial at 25°C is 40 to 100 kPa.

12. The kit formulation according to Claim 10, comprising a 51 to 300 mL vial containing the injectable composition according to Claim 1, wherein the internal atmosphere of the vial at 25°C is 0.5 to 39 kPa.

13. The kit formulation according to Claim 10, comprising (i) a compartment encapsulating the injectable composition according to Claim 1 in the form of a solid therein and (ii) a compartment encapsulating a physiological saline solution or a glucose solution therein, wherein the two compartments are constructed such that they can communicate with each other at use.

14. An injectable composition obtained by lyophilizing a solution of the composition according to Claim 1.

15. An injectable composition obtained by freezing a solution of the composition according to Claim 1.

## Patentansprüche

1. Injizierbare Zusammensetzung, die eine saure pharmazeutische Verbindung, Natriumcarbonat und Arginin umfasst, wobei wenigstens die saure pharmazeutische Verbindung fest ist,
wobei die saure pharmazeutische Verbindung ein Salz einer organischen Säure, ein Salz einer anorganischen Säure, ein organisches Säureaddukt oder ein anorganisches Säureaddukt von Cefotiam oder eine durch die Formel (I) dargestellte Verbindung ist: 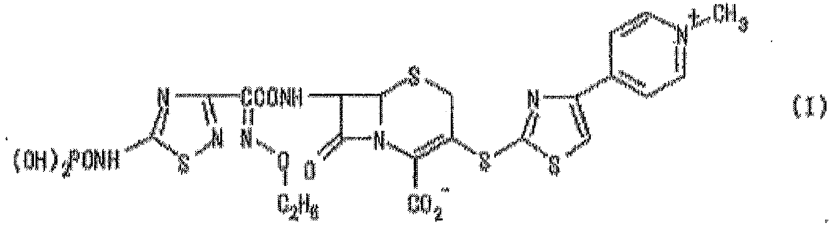 wobei die Gesamtmenge an Natriumcarbonat und Arginin 0,3 bis 6,2 Äquivalente pro einem Äquivalent der sauren pharmazeutischen Verbindung beträgt,
wobei die Menge an Natriumcarbonat 0,1 bis 3,4 Äquivalente pro einem Äquivalent der sauren pharmazeutischen Verbindung beträgt.

2. Injizierbare Zusammensetzung gemäß Anspruch 1, wobei es sich bei der sauren pharmazeutischen Verbindung um Cefotiam-Hydrochlorid handelt.

3. Injizierbare Zusammensetzung gemäß Anspruch 1, wobei der pH-Wert nach dem Auflösen 3,5 bis 9,5 beträgt.

4. Injizierbare Zusammensetzung gemäß Anspruch 1, wobei die Gesamtmenge an Natriumcarbonat und Arginin 0,07 bis 0,82 Gewichtsteile pro einem Gewichtsteil der sauren pharmazeutischen Verbindung beträgt.

5. Injizierbare Zusammensetzung gemäß Anspruch 1, wobei die Menge an Natriumcarbonat 0,01 bis 0,22 Gewichtsteile pro einem Gewichtsteil der sauren pharmazeutischen Verbindung beträgt.

6. Injizierbare Zusammensetzung gemäß Anspruch 1, wobei die Menge an Arginin 0,2 bis 2,8 Äquivalente pro einem Äquivalent der sauren pharmazeutischen Verbindung beträgt.

7. Injizierbare Zusammensetzung gemäß Anspruch 1, wobei die Menge an Arginin 0,06 bis 0,60 Gewichtsteile pro einem Gewichtsteil der sauren pharmazeutischen Verbindung beträgt.

8. Stechampullenzubereitung, die die injizierbare Zusammensetzung gemäß Anspruch 1 enthält.

9. Stechampullenzubereitung gemäß Anspruch 8, wobei die innere Atmosphäre bei 25 °C 40 bis 100 kPa beträgt.

10. Kitzubereitung, die die injizierbare Zusammensetzung gemäß Anspruch 1 enthält.

11. Kitzubereitung gemäß Anspruch 10, die eine 3- bis 50-ml-Stechampulle umfasst, welche die injizierbare Zusammensetzung gemäß Anspruch 1 enthält, wobei die innere Atmosphäre der Stechampulle bei 25 °C 40 bis 100 kPa beträgt.

12. Kitzubereitung gemäß Anspruch 10, die eine 51- bis 300-ml-Stechampulle umfasst, welche die injizierbare Zusammensetzung gemäß Anspruch 1 enthält, wobei die innere Atmosphäre der Stechampulle bei 25 °C 0,5 bis 39 kPa beträgt.

13. Kitzubereitung gemäß Anspruch 10, umfassend:
(i) ein Kompartiment, das die injizierbare Zusammensetzung gemäß Anspruch 1 in Form eines Feststoffs einkapselt, und
(ii) ein Kompartiment, das eine physiologische Kochsalzlösung oder eine Glucoselösung einkapselt,
wobei die zwei Kompartimente so aufgebaut sind, dass sie bei Verwendung miteinander kommunizieren können.

14. Injizierbare Zusammensetzung, die durch Lyophilisieren einer Lösung der Zusammensetzung gemäß Anspruch 1 erhalten wird.

15. Injizierbare Zusammensetzung, die durch Gefrierenlassen einer Lösung der Zusammensetzung gemäß Anspruch 1 erhalten wird.

## Revendications

1. Composition injectable comprenant un composé pharmaceutique acide, du carbonate de sodium et de l'arginine, dans laquelle au moins le composé pharmaceutique acide est solide, dans laquelle le composé pharmaceutique acide est un sel d'acide organique, un sel d'acide inorganique, un produit d'addition d'acide organique ou un produit d'addition d'acide inorganique de céfotiam, ou un composé représenté par la formule (I) : 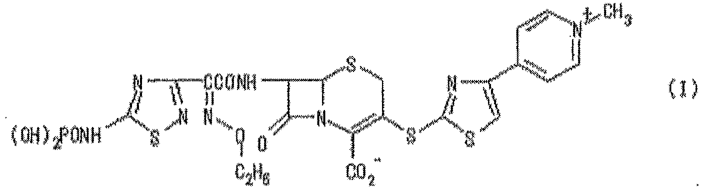 dans laquelle la quantité totale de carbonate de sodium et d'arginine est de 0,3 à 6,2 équivalents pour 1 équivalent du composé pharmaceutique acide,
dans laquelle la quantité de carbonate de sodium est de 0,1 à 3,4 équivalents pour 1 équivalent du composé pharmaceutique acide.

2. Composition injectable selon la revendication 1, dans laquelle le composé pharmaceutique acide est le chlorhydrate de céfotiam.

3. Composition injectable selon la revendication 1, dans laquelle le pH après dissolution est de 3,5 à 9,5.

4. Composition injectable selon la revendication 1, dans laquelle la quantité totale de carbonate de sodium et d'arginine est de 0,07 à 0,82 partie en poids pour 1 partie en poids du composé pharmaceutique acide.

5. Composition injectable selon la revendication 1, dans laquelle la quantité de carbonate de sodium est de 0,01 à 0,22 partie en poids pour 1 partie en poids du composé pharmaceutique acide.

6. Composition injectable selon la revendication 1, dans laquelle la quantité d'arginine est de 0,2 à 2,8 équivalents pour 1 équivalent du composé pharmaceutique acide.

7. Composition injectable selon la revendication 1, dans laquelle la quantité d'arginine est de 0,06 à 0,60 partie en poids pour 1 partie en poids du composé pharmaceutique acide.

8. Formulation pour flacon contenant la composition injectable selon la revendication 1.

9. Formulation pour flacon selon la revendication 8, dans laquelle l'atmosphère interne à 25 °C est de 40 à 100 kPa.

10. Formulation pour kit contenant la composition injectable selon la revendication 1.

11. Formulation pour kit selon la revendication 10, comprenant un flacon de 3 à 50 ml contenant la composition injectable selon la revendication 1, dans laquelle l'atmosphère interne du flacon à 25 °C est de 40 à 100 kPa.

12. Formulation pour kit selon la revendication 10, comprenant un flacon de 51 à 300 ml contenant la composition injectable selon la revendication 1, dans laquelle l'atmosphère interne du flacon à 25 °C est de 0,5 à 39 kPa.

13. Formulation pour kit selon la revendication 10, comprenant (i) un compartiment encapsulant la composition injectable selon la revendication 1 sous la forme d'un solide à l'intérieur et (ii) un compartiment encapsulant une solution saline physiologique ou une solution de glucose à l'intérieur, dans laquelle les deux compartiments sont construits de telle sorte qu'ils peuvent communiquer l'un avec l'autre au moment de l'utilisation.

14. Composition injectable obtenue par lyophilisation d'une solution de la composition selon la revendication 1.

15. Composition injectable obtenue par congélation d'une solution de la composition selon la revendication 1.
